# EUROPÄISCHE PATENTSCHRIFT

(11) **EP 0 407 475 B1**
(45) Veröffentlichungstag und Bekanntmachung des Hinweises auf die Patenterteilung: **02.02.1994**
(21) Anmeldenummer: 89908697.9
(22) Anmeldetag: 04.08.1989
(51) Int. Cl.: A61F 5/04

(54) **VORRICHTUNG ZUR ABSCHLIESSENDEN FIXIERUNG VON EXTREMITÄTEN**
DEVICE FOR ENSHEATHED FIXATION OF LIMBS
DISPOSITIF POUR LA FIXATION ENGAINEE DE MEMBRES

(30) Priorität: 05.08.1988 DE 3826704; 30.12.1988 DE 3844381
(43) Veröffentlichungstag der Anmeldung: 16.01.1991
(73) Patentinhaber: Habermeyer, Peter, Dr., D-70184 Stuttgart (DE)
(72) Erfinder: Habermeyer, Peter, Dr., D-70184 Stuttgart (DE)
(74) Vertreter: Richter, Werdermann & Gerbaulet
(86) Internationale Anmeldenummer: EP8900926
(87) Internationale Veröffentlichungsnummer: WO9001309

(56) Entgegenhaltungen:
- CH-A- 661 204
- DE-A- 2 018 605
- US-A- 4 724 627

## Beschreibung

Die Erfindung betrifft eine Vorrichtung zur Behandlung von Extremitätenfrakturen im Bereich von Unterschenkel, Oberschenkel, Unterarm und Oberarm.

Zur Behandlung von Extremitätenfrakturen werden üblicherweise Gipsverbände oder deren moderne Varianten in Form von Kunststoffverbänden angelegt. Das Anlegen derartiger Verbände erfordert nicht nur ein erhebliches Geschick und einen entsprechenden Aufwand für das Personal, sondern führt häufig auch dazu, daß durch Kanten oder Vorsprünge an der Innenseite des Gips- oder Plastikverbandes für den Patienten äußerst störende Druckstellen entstehen. Nach der Reposition der jeweiligen Fraktur ist der Patient der schmerzhaften Phase des Nachwickelns der Binden und des Wartens auf das Abbinden des Gipses ausgesetzt. Schließlich ist es bei den bekannten Gips- oder Plastikverbänden im Regelfall erforderlich, einen Wechsel dieses GiPs- oder Plastikverbandes vorzunehmen, wenn es im Laufe der Frakturbehandlung zu einer Abschwellung der betreffenden Gliedmaße kommt. Auch dies ist ein aufwendiger und für den Patienten wiederum unangenehmer Vorgang.

Aus der DE-A-2 018 605 ist eine Vorrichtung zum Ruhigstellen von Gliedmaßen bekannt, die als vakuumversteifbare, flexible Schiene ausgebildet ist. Eine solche flexible Schiene besteht dabei aus einer einen oder mehrere Hohlräume enthaltenden flexiblen Hülle. Die Hülle ist zur stufenfreien Arretierung mit Klettverschlüssen versehen. In dem Hohlraum oder den Hohlräumen dieser flexiblen Hülle sind kleine, leichte Körner oder Teilchen vorgesehen. Die flexible Hülle ist mit zumindest einem Ventil versehen, das mit einer Vakuumquelle verbunden werden kann. Durch Evakuierung des Hohlraums bzw. der Hohlräume läßt sich eine Verfestigung der flexiblen Hülle in Verbindung mit der in ihr enthaltenen Körner oder Teilchen erreichen.

Die durch diese Evakuierung der mit kleinen Körnern oder Teilchen gefüllten Hülle erzielbare Festigkeit der Stützanordnung reicht jedoch in den meisten Fällen nicht aus, um eine Stabilität und Steifigkeit zu erzielen, die Voraussetzung dafür ist, daß beispielsweise bei Frakturen die Relativlage von gegenseitig wieder ausgerichteten Knochenteilen gewährleistet werden kann. Außerdem ist aufgrund der Verletzbarkeit der flexiblen Hülle stets die Gefahr einer Beschädigung gegeben, die zu einem vollständigen Festigkeitsverlust und damit auch zu den daraus unmittelbar resultierenden Gefahren für den Patienten führen kann.

Aufgabe der Erfindung ist es, eine Vorrichtung der eingangs angegebenen Art in der Weise auszubilden, daß die Nachteile der bekannten Verbände und Stützanordnungen vermieden werden und eine einfach und schnell vornehmbare, zu keinerlei Druckstellen führende umschließende Fixierung von Extremitäten möglich ist, die praktisch unverzüglich wirksam ist. Außerdem sollte diese Vorrichtung eine hohe Stabilität und Festigkeit besitzen sowie problemfrei nachgestellt, einfach gelöst und auch sofort wieder neu angelegt werden können.

Diese Aufgabe wird durch die im Patentanspruch angegebenen Merkmale gelöst.

Aufgrund der an die jeweilige Extremität angepaßten Schalenstruktur der stabilen Kunststoffhülse kann bezüglich der jeweiligen Fraktur eine sichere Grobpositionierung erreicht werden, und es wird durch die an die jeweilige Extremität gut anmodellierbaren Manschettenkissen im Zusammenwirken mit der stabilen Kunststoffhülse eine Feinpositionierung ermöglicht.

Durch den Evakuiervorgang wird unter Beseitigung kleinflächiger störender Druckstellen eine Vergrößerung des lichten Innendurchmessers der aus der stabilen Kunststoffhülse und den verfestigten Manschettenkissen bestehenden Vorrichtung erzielt, was zur Folge hat, daß zwischen der jeweiligen Extremität und der Umhüllung ein geringer Spalt entsteht, der wiederum aufgrund der stufenfreien Arretierbarkeit der aufklappbar ausgebildeten und mit Klettverschlüssen versehenen Kunststoffhülse auf einen optimal kleinen Wert, und zwar auch nachträglich eingestellt werden kann.

Das Manschettenkissen besteht aus einem für Röntgenstrahlen durchlässigen Material, insbesondere aus einem im wesentlichen nicht dehnbaren Kunststoff-Folienmaterial in hautfreundlicher Ausführung oder entsprechender hautseitiger Ausrüstung.

Durch die Wahl eines im wesentlichen nicht dehnbaren Folienmaterials wird wesentlich zur Verfestigung der evakuierten Hülse beigetragen, da die eng an den Füllkörperteilchen anliegende Folie nicht gedehnt werden kann und damit in Verbindung mit den zwischen den Füllkörpern geschaffenen Reibschlußkontakten ein praktisch starres Gebilde entsteht.

Die Füllkörperteilchen des flachen, zu einer Hülse verformbaren Manschettenkissens bestehen zumindest zum Teil aus einander flächig überlappenden und im aneinandergepreßten Zustand kraftschlüssig verbundenen Teilchen, die vorzugsweise zumindest im wesentlichen inkompressibel sind. Hinsichtlich der verwendbaren Füllkörperteilchen besteht eine große Auswahl, aber es ist bei dieser Auswahl auch darauf zu achten, daß möglichst leichte Teilchen Verwendung finden, die aber in ihrem Zusammenwirken, d.h. im aneinandergepreßten und zwischen den Wänden des Manschettenkissens eingeschlossenen Zustand zu einer hohen Gesamtfestigkeit führen.

In dem Manschettenkissen kann zumindest ein den Durchtritt von Extensionsdrähten, Schläuchen und dergleichen ermöglichender Perforationsbereich mit vakuumdichten Rändern vorgesehen sein, so daß ohne Schwierigkeiten auch eine Drahtextensionsbehandlung im Bereich des Unterschenkels erfolgen kann.

Vorzugsweise besitzt die Kunststoffhülse eine innere Beschichtung in Form einer hartelastischen Polsterung, welche die unterschiedlich großen Raumbereiche zwischen Innenhülse und Außenhülse füllt und gleichzeitig bei Aufprallbelastungen Stoßpufferfunktion übernimmt.

Die erfindungsgemäße Vorrichtung kann auch nach Art eines langen Oberschenkelgipses oder als stabile, sich vom Sprunggelenk bis zum Oberschenkelansatz erstreckende Hülse ausgebildet werden, und es bereitet auch keine Schwierigkeit, eine Ausgestaltung nach Art eines Unterschenkelliege- oder -gehgipses vorzunehmen.
Weitere Anwendungen sind beispielsweise möglich bei Radiusfrakturen zur Ruhigstellung von Hand und Unterarm sowie als Oberarm-Brace zur Ruhigstellung von Oberarmfrakturen, und es ist natürlich auch eine Verwendung nach Art eines Oberarmgipses zur Ruhigstellung von Oberarm, Unterarm und Handgelenk möglich.

Die Erfindung wird anhand eines Ausführungsbeispiels und unter Bezugnahme auf die Zeichnung näher erläutert; in der Zeichnung zeigt:
- Fig. 1: eine schematische Längsschnittdarstellung eines mit einer Vorrichtung nach der Erfindung versehenen Unterschenkels,
- Fig. 2: eine schematische Teil-Querschnittsdarstellung einer Ausführungsform der Vorrichtung nach der Erfindung, und
- Fig. 3: eine Schnittdarstellung entsprechend der Linie A-A von Fig. 1.

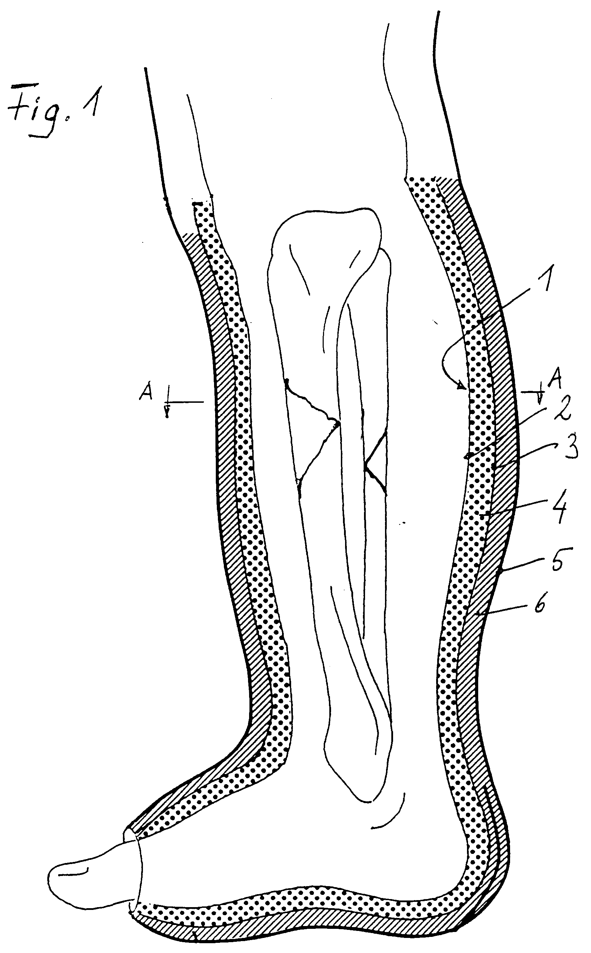

Fig. 1 zeigt einen Unterschenkel, an dem anstelle eines herkömmlichen Gipsverbandes eine Vorrichtung nach der Erfindung angebracht ist.
Diese Vorrichtung umfaßt ein zu einer Hülse geformtes Manschettenkissen 1, das aus einer Innenwand 2 und einer Außenwand 3 besteht, die vakuumdicht ausgebildet und an ihren Randbereichen vakuumdicht miteinander verbunden, insbesondere verschweißt sind, so daß ein Innenraum entsteht, der mit einer Vielzahl einzelner Füllkörperteilchen angefüllt ist. Auf diese Weise entsteht ein sehr flaches, kissenartiges und sehr schmiegsames Gebilde, das um den jeweiligen Gliedmaßenabschnitt herumgelegt und insbesondere mittels eines Klettverschlusses bündig und formschlüssig arretiert werden kann.

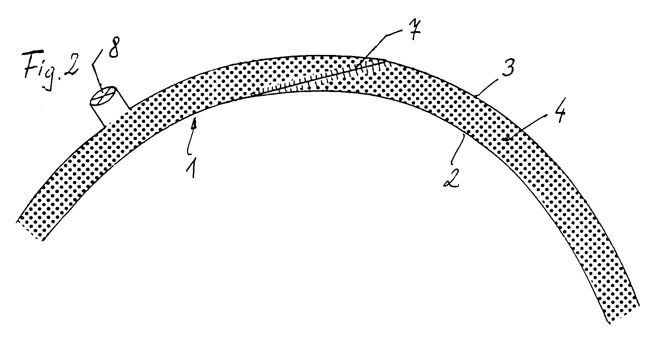

Die Teilschnittdarstellung nach Fig. 2 zeigt das Manschettenkissen 1 im Bereich seiner Verbindung zu einem Hülsengebilde, wobei die Randbereiche des Manschettenkissens mit Klettverschlußelementen 7 versehen sind, so daß durch entsprechende Überlappung das Hülsengebilde auch schon dann in seiner Grundform festgelegt werden kann, wenn der Absaugvorgang noch nicht stattgefunden hat. Um eine Evakuierung des Raumes zwischen den beiden Wandungen 2 und 3 zu ermöglichen, ist zumindest ein Evakuierungsventil 8 vorgesehen.

Nach der Arretierung des Plastisch gut anmodellierten Hülsengebildes und nach genauer Einrichtung des frakturierten Unterschenkels und der Röntgenkontrolle wird durch Anschalten einer Absaugpumpe innerhalb sehr kurzer Zeit der erforderliche hohe Unterdruck im Manschettenkissen erzeugt, der dazu führt, daß die Füllkörperteilchen untereinander aufgrund des Differenzdrucks zur Atmosphäre durch die Wandungen 2 und 3 fest aneinander gepreßt werden, so daß aufgrund des Kraft- und Formschlusses zwischen den Wandungen 2 und 3 und den Füllkörperteilchen ein steifes Gebilde entsteht, das den gebrochenen Unterschenkel schient und stabilisiert.

Zum Schutz dieses unter Vakuum stehenden Gebildes und zur weiteren Stabilisierung wird eine äußere Kunststoffhülse 5 angelegt, die in etwa die Form des Unterschenkels aufweist und schalenartig ausgebildet oder so flexibel ist, daß über einen ventralen Schlitz ein Anlegen möglich ist. An der Innenseite der äußeren Kunststoffhülse 5 ist eine hartelastische Polsterung 6 vorgesehen, welche die Zwischenräume zwischen der verfestigten Innenhülse 1 und der Außenhülse 5 ausfüllt und zugleich als Stoßpuffer bei Aufprallbelastungen wirkt und z.B. beim Gehen Widerstand leistet.

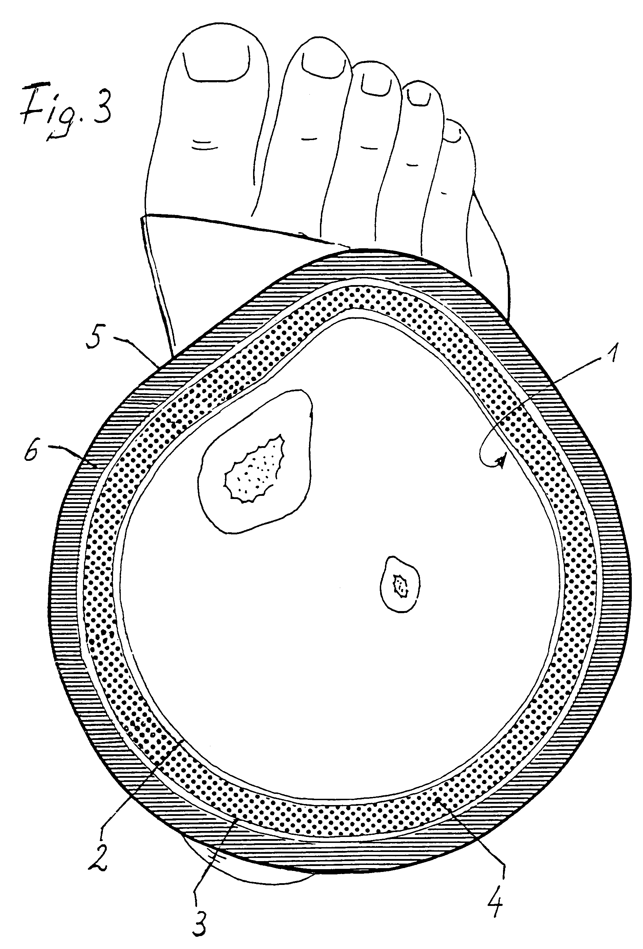

Die Schnittansicht nach Fig. 3 läßt erkennen, daß von der verfestigten Innenhülse 1 auf den Unterschenkel kein störender, insbesondere kleinflächiger Druck ausgeübt werden kann, da die Verfestigung dieser Hülse beim Anlegen einer Unterdruckquelle zu einer Vergrößerung des lichten Innendurchmessers führt, was zur Folge hat, daß zwischen dem Unterschenkel und der verfestigten Innenhülse ein geringer Spalt entsteht.

Neben den bereits erwähnten Vorteilen ist darauf hinzuweisen, daß die Vorrichtung nach der Erfindung sterilisierbar ausgeführt werden kann, damit häufig wiederverwendbar ist und zu einer Senkung der Behandlungskosten beiträgt. Es ist insbesondere auch eine Verwendung bei der Behandlung offener Frakturen möglich, weil ein Verbandswechsel Problemlos durch Aufhebung des Unterdrucks möglich ist und damit die Wunde gut zugänglich wird. Ein erneutes Anlegen ist innerhalb kurzer Zeit problemlos möglich. Dies führt auch dazu, daß sogar eine Abnahme des Verbandes aus hygienischen Gründen, z.B. zum Duschen, vertretbar ist und vor allem auch nach Lockerung der Klettverschlüsse und dann erfolgende erneute Evakuierung eine engere Anlegung des Verbandes möglich ist, wenn es im Verlaufe der Frakturbehandlung zu einer Abschwellung des zu behandelnden Gliedes kommt. Der sonst erforderliche Gipswechsel entfällt auf diese Weise.

Die Erfindung ist vorteilhaft im Bereich der konservativen Behandlung von Brücken sowie Kontusionen und Distorsionen der oberen und unteren Extremität im Fachgebiet Chirurgie, Unfallchirurgie und Orthopädie verwendbar.

## Patentansprüche

1. Vorrichtung zur Behandlung von Extremitätenfrakturen im Bereich von Unterschenkel, Oberschenkel, Unterarm und Oberarm, bestehend aus
a) einer der Form der jeweiligen Extremität angepaßten stabilen Kunststoffhülse (5),
b) die für ein Anlegen an die jeweilige Extremität schalenartig aufklappbar ausgebildet und zur stufenfreien Arretierung mit Klettverschlüssen versehen ist, sowie
c) einem innerhalb der Kunststoffhülse (5) angeordneten, an die jeweilige Extremität hülsenförmig anmodellierbaren, vakuumdicht, doppelwandig ausgebildeten, mit zumindest einem Evakuierungsventil versehenen, zu einer Hülse verformbaren, vakuumversteifbaren Manschettenkissen (1),
d) dessen zwischen den beiden Wänden gelegener Innenraum mit einer Vielzahl relativ zueinander beweglicher Füllkörper versehen ist.

## Claims

1. Device for the treatment of extremity fractures within the area of the lower leg, thigh, forearm and upper arm, comprising
a) a stable plastic sheath (5) adapted to the configuration of the respective extremity;
b) which, for being applied to the respective extremity so as to be able to be folded out in a shell-shaped manner and, for the continuous retention, is provided With Velcro fasteners, as well as
c) a vacuum-stiffenable sleeve-like padding (1) disposed inside the plastic sheath (5), which can be modelled onto the respective extremity in a sheath-like fashion, constructed so as to be vacuum-tight and double-walled, provided With at least one evacuation valve and deformable into a sheath;
d) whose interior located between the two walls is provided with a large number of filler bodies which are movable relative to each other.

## Revendications

1. Dispositif pour traiter les fractures d'extrémités dans la zone des bas de jambe, cuisses, bras, avant-bras, composé
a) d'une gaine de matière plastique (5) stable adaptée à la forme de l'extrémité respective,
b) qui est configurée en pouvant se replier comme une enveloppe pour adhérer à l'extrémité respective et qui est pourvue de fermetures auto-agrippantes pour un blocage non gradué ainsi que
c) d'un coussin en manchette (1) placé à l'intérieur de la gaine de matière plastique (5), qui peut être modelé en forme de gaine sur l'extrémité respective, étanche au vide, configuré à double paroi, pourvu d'au moins une vanne de vide, déformable pour former une gaine et pouvant être raidi par le vide
d) dont l'espace intérieur, situé entre les deux parois, est pourvu d'une multitude de corps de remplissage mobiles les uns par rapport aux autres.
